(19) Europäisches Patentamt
European Patent Office
Office européen des brevets

(11) **EP 3 686 154 A1**

(12) **EUROPÄISCHE PATENTANMELDUNG**

(43) Veröffentlichungstag:
**29.07.2020  Patentblatt 2020/31**

(51) Int Cl.:
*C01B 3/34* (2006.01)   *C07C 29/00* (2006.01)
*C25B 1/00* (2006.01)   *C07C 31/04* (2006.01)

(21) Anmeldenummer: **19153501.2**

(22) Anmeldetag: **24.01.2019**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Siemens Aktiengesellschaft**
**80333 München (DE)**

(72) Erfinder: **Ungerer, Markus**
**91083 Baiersdorf (DE)**

(54) **VERFAHREN ZUM BETREIBEN EINER ANLAGE ZUR SYNTHESE EINES CHEMISCHEN PRODUKTS**

(57)    Die Erfindung betrifft ein Verfahren zum Betreiben einer Anlage (10) zur Synthese eines chemischen Produkts (12), mit wenigstens zwei Empfangsstellen (14, 16), an welchen jeweils wenigstens ein Reaktant empfangbar ist, und wenigstens einem Reaktionsbereich (18), innerhalb welchen das Produkt (12) durch wenigsten eine Reaktion aus dem wenigstens einen Reaktanten erzeugt wird, wobei die Anlage (10) ausgelegt ist für einen bestimmten Durchsatz, bei welchem pro Zeiteinheit eine erste Menge an Reaktant in dem Reaktionsbereich (18) reagieren kann, mit den Schritten:

- Leiten einer zweiten gegenüber der ersten Menge geringeren Menge an Reaktant von einer ersten der Empfangsstellen (14) in den Reaktionsbereich (18) (S1);
- Bestimmen einer durch eine zweite der Empfangsstellen (16) empfangbare Bereitstellungsmenge an Reaktant (S2); und
- Leiten einer dritten Menge an Reaktant in Abhängigkeit von der Bereitstellungsmenge des Reaktanten von der zweiten Empfangsstelle (16) in den Reaktionsbereich (18) (S3).

FIG 2

EP 3 686 154 A1

**Beschreibung**

Die Erfindung betrifft ein Verfahren zum Betreiben einer Anlage zur Synthese eines chemischen Produkts.

Energie, insbesondere elektrische Energie, wird vermehrt aus regenerativen Energiequellen gewonnen, welche somit den Anteil an fossilen Energieträgern zurückdrängen. Diese sogenannte Energiewende hat unter anderem zum Ziel, eine Emission von Treibhausgasen, insbesondere $CO_2$, zu verringern. Durch die Reduzierung der Emission von Treibhausgasen soll einer Klimaerwärmung entgegengewirkt werden. Damit die Emissionsreduzierung besonders groß wird, um beispielsweise die Klimaerwärmung auf weniger als 2 °C zu begrenzen, sollte nicht nur der Stromsektor neu gestaltet werden. Zusätzlich ist dazu auch beispielsweise eine Neugestaltung des Wärmesektors und des Mobilitätssektors vorteilhaft. Das heißt die genannten Sektoren sollten vorteilhafterweise mit erneuerbaren Energien durchdrungen werden.

Eine Möglichkeit hierfür, vor allem im Bereich der Mobilität, ist eine Verwendung von synthetischen Kraftstoffen, wie beispielsweise Methanol ($CH_3OH$). Die Herstellung bzw. Erzeugung eines synthetischen Kraftstoffs erfolgt in der Regel durch eine chemische Synthese bzw. Reaktion, bei welcher aus wenigstens einem Reaktanten bzw. einem Edukt der Kraftstoff als Produkt gewonnen wird. Bei kohlenstoffbasierten Kraftstoffen werden als Reaktanten typischerweise Wasserstoff und Kohlenmonoxid verwendet, es sind aber auch andere Reaktanten denkbar.

Um den Reaktant, welcher insbesondere gasförmig in einem Synthesegas vorliegt, zu gewinnen, stellt beispielsweise eine Elektrolyse einen besonders vorteilhaften technologischen Prozess dar. So kann bei einer Wasserelektrolyse Wasserstoff erzeugt werden, aus welchem mit Kohlenmonoxid, das über eine umgekehrte Wasser-Gas-Shift-Reaktion (RWGS-Reaktion) gewonnen wird, oder beispielsweise durch direkte Synthese aus Kohlendioxid und Wasserstoff der Kraftstoff synthetisiert werden. Alternative ist beispielsweise eine Kohlendioxidelektrolyse, durch welche eine direkte Erzeugung von Kohlenmonoxid in Verbindung mit einer Wasserelektrolyse zum Erzeugen des Synthesegases kombiniert werden kann.

Anlagen, insbesondere großtechnischer Natur, wie beispielsweise Raffinerien oder chemische Synthesen, haben in der Regel eine Entwicklung hinter sich, welche, aufgrund kontinuierlich verfügbarer Energie (bzw. Massenstrom)von fossilen Energieträgern, stets auf einen möglichst kontinuierlichen Betrieb hin optimiert wurden, um insbesondere hohe Umsätze und lange Standzeiten zu realisieren. Aufgrund einer Volatilität in der Stromerzeugung aus erneuerbaren Quellen kann ohne geeignete Maßnahmen, wie beispielsweise die Verwendung von Stromspeichern oder Gasspeichern, dieser kontinuierliche Betrieb nicht aufrechterhalten werden, wodurch signifikante Mehrkosten entstehen würden.

Aufgabe der vorliegenden Erfindung ist es daher, ein Verfahren zum Betreiben einer Anlage, insbesondere einer Syntheseanlage, wie beispielsweise einer Raffinerie, bereitzustellen, sodass durch die Anlage eine Synthese eines chemischen Produkts, insbesondere unter der Einbeziehung regenerativer Energieträger, derart erfolgen kann, dass die Synthese auch ohne oder mit einem besonders geringen Einsatz von Stromspeichern und/oder Gasspeichern besonders effizient und kostengünstig ist.

Diese Aufgabe wird erfindungsgemäß durch ein Verfahren gemäß dem Patentanspruch 1 gelöst. Vorteilhafte Ausgestaltungen mit zweckmäßigen Weiterbildungen der Erfindung sind in den übrigen Ansprüchen angegeben.

Durch das erfindungsgemäße Verfahren wird eine Anlage zur Synthese eines chemischen Produkts, und somit insbesondere eine Syntheseanlage beispielsweise für die Methanolsynthese, betrieben. Die Anlage weist wenigstens zwei Empfangsstellen auf, an welchen durch das Verfahren jeweils wenigstens ein Reaktant, insbesondere mittels eines Synthesegases, empfangbar ist. Innerhalb wenigstens eines Reaktionsbereichs der Anlage wird das Produkt, beispielsweise Methanol, durch wenigstens eine Reaktion aus dem wenigstens einen Reaktanten erzeugt. Die Anlage ist für einen bestimmten Durchsatz, bei welchem pro Zeiteinheit eine erste Menge an Reaktant in dem Reaktionsbereich reagieren kann, ausgelegt. Die erste Menge entspricht somit vorteilhafterweise 100 % der Menge, welche durch die Anlage innerhalb der Zeiteinheit verarbeitbar ist.

Der Reaktant ist eine Substanz, die während eines Ablaufs der, insbesondere chemischen, Reaktion verbraucht wird. Der Reaktant kann sowohl als Element bzw. als Reinstoff aber auch als chemische Verbindung ausgebildet sein. Aus dem Reaktant wird durch die, insbesondere chemische, Reaktion das Produkt hergestellt bzw. erzeugt. Der Reaktant wird auch als Edukt oder Ausgangsstoff bezeichnet. Das Synthesegas kann wenigstens einen Reaktanten und somit insbesondere explizit einen Reaktanten oder mehrere Reaktanten umfassen. Somit kann das Synthesegas der Reaktant selbst sein oder mehrere Reaktanten umfassen.

Damit die Anlage für die Synthese des Produkts besonders vorteilhaft betrieben werden kann, sodass insbesondere der wenigstens eine Reaktant bzw. das Edukt beispielsweise vorteilhaft unter der Verwendung erneuerbaren Energien gewonnen werden kann, umfasst das Verfahren die folgenden Schritte:
In einem ersten Schritt des Verfahrens erfolgt ein Leiten einer zweiten gegenüber der ersten Menge geringeren Menge an Reaktant von einer ersten der Empfangsstellen in den wenigstens einen Reaktionsbereich. Bei der Empfangsstelle kann es sich beispielsweise, insbesondere für den Fall, dass für das Verfahren ein Synthesegas verwendet wird beispielsweise um das Ende einer Pipeline und/oder ein Ventil handeln, an welchem der Reaktant beispielsweise durch Öffnen des Ventils empfangen werden kann bzw. wird an der Empfangsstelle der Reaktant bereitgestellt, sodass er von dort, beispielsweise durch eine Leitung bzw. ein Rohr, in den Reaktionsbereich geleitet bzw. gefördert werden kann. Dabei ist vorteilhafterweise insbesondere ein kontinuierliches Leiten bzw. ein Einleiten des Reaktanten an der Empfangsstelle möglich, sodass die wenigstens eine in dem Reaktionsbereich stattfindende Reaktion insbesondere kontinuierlich aufrechterhalten werden kann. Dabei ist eine Vielzahl unterschiedlicher Ausführungsform der Empfangsstellen realisierbar. Ferner ist das Leiten bzw. Fördern des Reaktanten in den Reaktionsbereich, welcher insbesondere beispielsweise als Reaktor oder Reaktorkammer und somit beispielsweise als Behälter ausgebildet ist, nicht auf das genannte Rohr bzw. die Leitung beschränkt, sondern das Leiten kann auch auf alternative Weise realisiert sein. Die Anlage ist insbesondere für einen kontinuierlichen Betrieb ausgelegt, sodass etwaige Verluste bei einer Inbetriebnahme der Anlage oder bei einem Abschalten der Anlage möglichst vermieden werden können, daher ist die Empfangsstelle vorteilhafterweise für das kontinuierliche Bereitstellen bzw. Empfangen des Reaktanten ausgebildet und das Leiten kann kontinuierlich durch die Anlage bzw. das Verfahren durchgeführt werden.

Unter der zweiten Menge ist ebenfalls eine Menge pro Zeiteinheit an Reaktant zu verstehen, welche pro Zeiteinheit in den Reaktorbereich geleitet wird. Die erste Menge bestimmt quasi den Durchsatz, welcher insbesondere in einem Betriebsmodus der Anlage realisiert werden kann, bei welchem die Anlage derart betrieben wird, dass pro Zeiteinheit eine besonders große Menge an Produkt hergestellt werden kann, ohne dass sich die Anlage beispielsweise aufgrund von Wärmeentwicklung in einem kritischen Zustand befindet. Somit entspricht die erste Menge im Wesentlichen 100 % Ausbeute der Anlage bzw. 100 % der Menge, welche durch die Anlage innerhalb der Zeiteinheit verarbeitbar ist. Die zweite Menge ist nun gegenüber der ersten Menge verringert, sodass beispielsweise die zweite Menge nur 80% der ersten Menge umfasst und somit 80 % Ausbeute der Anlage innerhalb der Zeiteinheit entsprechen. Dabei sind beliebige andere Prozentsätze realisierbar. Das heißt die Anlage wird durch das Verfahren derart über die erste Empfangsstelle mit Reaktant versorgt, dass prinzipiell über eine weitere der Empfangsstellen, insbesondere jederzeit, während des Betriebs der Anlage, bei welchem die Reaktion in dem Reaktionsbereich stattfindet bzw. insbesondere aufrechterhalten wird, eine zusätzliche Menge an Reaktant in den Reaktionsbereich geleitet werden kann.

Daher erfolgt in einem zweiten Schritt des Verfahrens ein Bestimmen einer durch eine zweite der Empfangsstellen empfangbare Bereitstellungsmenge an Reaktant. Dabei ist an der zweiten Empfangsstelle der Reaktant, insbesondere beispielsweise nicht kontinuierlich empfangbar, da dieser durch eine volatile Quelle an der Empfangsstelle bereitgestellt wird. Dabei kann das bestimmen der, insbesondere aktuell, verfügbaren Menge, welche der Bereitstellungsmenge entspricht, durch eine entsprechend ausgebildete Mengenbestimmungsvorrichtung erfolgen. Da der Reaktant beispielsweise mittels Strom aus einer erneuerbaren Energiequelle, wie beispielsweise Photovoltaik oder Windkraft, erzeugt wird, kann dieser somit in Abhängigkeit, beispielsweise der Sonnenstrahlung oder eines Windes nicht ständig verfügbar sein. Um die Anlage besonders vorteilhaft betreiben zu können und beispielsweise die zweite Menge entsprechend abzustimmen, wird die Bereitstellungsmenge bestimmt.

Somit erfolgt in einem dritten Schritt des erfindungsgemäßen Verfahrens ein Leiten einer dritten Menge an Reaktant in Abhängigkeit von der Bereitstellungsmenge des Reaktanten von der zweiten Empfangsstelle in den Reaktionsbereich, sodass eine Summenmenge, insbesondere pro Zeiteinheit, umfassend die zweite Menge Reaktant aus der ersten Empfangsstelle und die dritte Menge Reaktant aus der zweiten Empfangsstelle in den Reaktionsbereich geleitet wird. Mit anderen Worten wird die in den Reaktionsbereich geleitete erste Menge mit der zweiten Menge beaufschlagt, sodass die Summenmenge größer der ersten Menge ist, sodass insbesondere die Ausbeute des durch die Anlage erzeugten Produkts pro Zeiteinheit im Vergleich zu der Ausbeute, welche nur durch die erste Menge erreichbar ist, steigt. Somit wird auf besonders vorteilhafte Weise der an der zweiten Empfangsstelle abrufbare Reaktant in die Synthese bzw. die Erzeugung des Produkts miteinbezogen.

Dabei können die Mengen, insbesondere die zweite Menge und die dritte Menge, jeweils als Volumenstrom pro Zeiteinheit aufgefasst werden, insbesondre für den Fall das der Reaktant als Fluid und somit beispielsweise gasförmig vorliegt bzw. geleitet wird.

Ist beispielsweise der an der ersten Empfangsstelle empfangbare Reaktant derart bereitgestellt, dass dieser in quasi beliebigen Mengen dort kontinuierlich zur Verfügung steht, beispielsweise da der Reaktant durch ein Verfahren bereitgestellt wird, welches beispielsweise den Reaktanten mittels fossilen und somit kontinuierlich verfügbaren Energieträgern erzeugt, und der an der zweiten Empfangsstelle empfangbare Reaktant ein Reaktant, welcher beispielsweise in seiner Menge durch die Verfügbarkeit an erneuerbarer Energie abgerufen werden kann, kann die Auslastung der Anlage in Abhängigkeit durch die an der zweiten Empfangsstelle verfügbare Bereitstellungsmenge getriggert werden.

Mit weiteren anderen Worten kann für das erfindungsgemäße Verfahren die Anlage so ausgelegt werden, dass sie eine Menge x an Produkt, beispielsweise Methanol, insbesondere pro einer bestimmten Zeiteinheit, erzeugen könnte. Diese Menge entspricht 100% und somit einem durch die Anlage erreichbaren Durchsatz. Eine weitere Anlage, welche beispielsweise den Reaktant, insbesondere in Form eines Synthesegases aus fossilen Rohstoffen erzeugt, beispielsweise ein Dampfreformer (auch Steamreformer) für Erdgas, erzeugt nun beispielsweise 80 % der Kapazität der Anlage und kann diese an der ersten Empfangsstelle bereitstellen. Für den Reaktanten, welcher an der zweiten Empfangsstelle empfangbar ist und welcher beispielsweise als ein erneuerbar erzeugtes Synthesegas ausgebildet ist, ergibt sich somit

3

ein Fenster, welches 20 % der, insbesondere maximalen, Kapazität der Anlage entspricht. Somit kann es sich ergeben, dass die Anlage ohne erneuerbare Energien nicht komplett ausgelastet ist, wodurch unter Verwendung von erneuerbare Energie die Anlage mittels des erfindungsgemäßen Verfahrens besonders vorteilhaft betrieben werden kann.

Dabei liegt der Erfindung die Erkenntnis zugrunde, dass Anlagen, wie beispielsweise eine Methanolsyntheseanlage, welche insbesondere als Anlage der Großindustrie ausgelegt ist, nahezu ausschließlich auf der Verwendung von Reaktant bzw. Reaktanten, welche als Synthesegas vorliegen können, aus fossilen Energiequellen bzw. Quellen wie beispielsweise Kohle oder Erdgas gespeist wurden, wodurch ein kontinuierlicher Prozess gewährleistet werden konnte. Für den Fall, dass die Synthese bzw. die Reaktion mit fluktuierendem Aufkommen des Reaktanten bzw. des Synthesegases betrieben werden musste, mussten bisher entsprechende Speicher bzw. Zwischenspeicher vorgehalten werden, beispielsweise ein Stromspeicher, insbesondere vor beispielsweise einer zur Erzeugung des Reaktanten durchzuführenden Elektrolyse oder einem Gasspeicher, welcher das durch die Elektrolyse erzeugte Synthesegas speichern konnte, sodass die Anlage darauf folgend die Reaktion bzw. Synthese kontinuierlich aufrechterhalten konnte. Dabei kann die Anlage eine Methanolanlage für die Synthese von Methanol als Produkt sein. Die Anlage kann beispielsweise auch derart aus gebildet sein, dass durch sie beispielsweise ein beliebiger Kohlenwasserstoffe als Produkte erzeugt werden kann. Es können, bei entsprechender Ausgestaltung der Anlage, aber auch jegliche andere Synthesen mittels des erfindungsgemäßen Verfahrens betrieben werden, beispielsweise die Fischer-Tropsch-Synthese. Die Anlage bzw. die in der Anlage stattfindende Reaktion bzw. Synthese kann in der Regel in einem begrenzten Rahmen und mit einer begrenzten Lastrampe in ihrer Auslastung variiert werden, wodurch die Anlage beispielsweise mit der zweiten Menge an Reaktanten statt der ersten Menge an Reaktanten betrieben werden kann. Das gilt auch für eine Erzeugung des Reaktanten, insbesondere in Form von Synthesegas, aus fossilen Quellen bzw. Energiequellen. Der Rahmen innerhalb welchem die Laständerung bzw. dessen Lastrampe stattfinden kann, wird durch die Größe der Anlage, also die Anlagengröße, definiert, da in der Regel das Ausmaß der durchführbaren Laständerung relativ zur Anlagengröße ist. Durch das erfindungsgemäße Verfahren wird ein Fenster geschaffen, welches durch die, insbesondere kurzzeitige, Produktion von Reaktant aus erneuerbaren Energiequellen bzw. die Bereitstellung des Reaktanten genutzt werden kann. Dabei kann das Fenster beispielsweise derart erzeugt werden, dass die erste Empfangsstelle bzw. eine der ersten Empfangsstelle zugeordnete Reaktantenquelle vorteilhafterweis so dimensioniert wird, dass die Anlage quasi in Bezug auf die erste Empfangsstelle und somit beispielsweise insbesondere in Bezug auf eine fossile Reaktantenquelle überdimensioniert, insbesondere bezüglich ihres Reaktionsbereichs, ausgebildet ist und somit Kapazitätsreserven für das Leiten der dritten Menge Reaktant von der zweiten Empfangsstelle in den Reaktionsbereich aufweist.

Die Anlage, welche insbesondere als Anlage für die Methanolsynthese ausgebildet ist, ist vorteilhafterweise eine bereits bestehende konventionelle Anlage und in der Regel stets auf einen möglichst kontinuierlichen Betrieb hin optimiert, um hohe Umsätze und lange Standzeiten zu realisieren. Dadurch ergibt sich eine mangelnde Flexibilität, da beispielsweise beim Anfahren bzw. Hochfahren und Herunterfahren der Anlage Druckwechsel und/oder Temperatursprünge durchgeführt werden müssen, welche die Lebensdauer beispielsweise eines in dem Reaktionsbereich verwendeten Katalysators deutlich verringern könnten. Vorrichtungen beispielsweise für eine Elektrolyse zur Erzeugung des Reaktanten aus erneuerbaren Energiequellen sind häufig für die Volatilität der Stromerzeugung aus erneuerbaren Energiequellen besser geeignet, sodass für beispielswiese eine Elektrolyse zur Erzeugung des Reaktanten eine besonders große Flexibilität bzw. Agilität aufweisen kann, um der fluktuierenden Verfügbarkeit von Strom aus erneuerbaren Energiequellen, wie beispielsweise Photovoltaik oder Windkraft, gerecht zu werden.

Durch das erfindungsgemäße Verfahren ergibt sich nun der Vorteil, dass insbesondere die dritte Menge des Reaktanten, welche an der zweiten Empfangsstelle empfangen wird, flexibel, zumindest innerhalb eines gewissen Fensters, sein kann. Wird die zweite Empfangsstelle beispielsweise aus einer erneuerbaren Quellen gespeist, kann diese flexibel betrieben werden, da die erste Menge beispielsweise durch einen kontinuierlich betriebenen fossilen Feed bereitgestellt wird und somit die relativen Schwankungen, insbesondere innerhalb der empfangbaren Mengen, kleiner sind. Daraus ergibt sich beispielsweise als weiterer Vorteil eine besonders effiziente Einbindung erneuerbarer Energien in diese Synthese bzw. Reaktion. Ferner ergibt sich durch das erfindungsgemäße Verfahren der Vorteil, dass dadurch eine, insbesondere intelligente, Kombination der Nutzung von bestehenden Anlagen mit bestehenden Rohstoffen bzw. Eduktströmen, insbesondere in Form von an der ersten Empfangsstelle bereitgestellten Reaktanten und neuen, erneuerbar produzierten Eduktströmen, wie dem an der zweiten Empfangsstelle mit der Bereitstellungsmenge bereitgestellten Reaktanten ergibt, sodass die Anlage derart betrieben werden kann, dass diese an der Energiewende partizipiert. Somit ist ein weiterer Vorteil des erfindungsgemäßen Verfahrens ein besonders ökologischer Betrieb der Anlage.

Ein weiterer Vorteil des Verfahrens, welcher durch die geleitete dritte Menge in den Reaktionsbereich, welche insbesondere aus erneuerbaren Energien und somit grün erzeugten Reaktanten umfasst, kann damit in Kombination mit dem Einleiten der zweiten Menge, welche insbesondere im Vergleich zu der dritten Menge größer als diese ist, die Anlage kontinuierlich betrieben werden und gleichzeitig ein nur flexibel erzeugbarer Reaktant, insbesondere relativ zu seiner Erzeugungsmenge mit besonders großem Mengenanteil, durch die Anlage in das Produkt synthetisiert werden. So können durch das Verfahren die Nachteile sowohl einer unflexiblen Synthese als auch beispielsweise einer unvollständigen Umsetzung des Reaktanten kompensiert werden.

Durch das Verfahren können somit, insbesondere besonders große Anlagen besonders vorteilhaft betrieben werden, da es sich bei besonders großen Anlagen bezüglich der für die Anlage auszugebenden Investitionen, insbesondere in Form des CAPEX (Captial Expenditure) von Vorteil ist, da dieser in der Regel gemäß der folgenden Gleichung mit einem Exponenten n von ca. 0,7 skaliert:

$$CAPEX_1 \; / \; CAPEX_0 = (Kapazität_1/Kapazität_0)^n$$

Dabei entspricht die Kapazität dem durch die Anlage erzielbaren Durchsatz, welcher durch die erst Menge an Reaktant ausgedrückt werden kann. Das erfindungsgemäße Verfahren ist somit insbesondere für ein Übergangsszenario, welches die Energiewende darstellt, besonders geeignet, da die Synthese des Produkts, insbesondere Methanol oder andere Verbindungen, die beispielsweise zur Herstellung als Reaktant, insbesondere in Form von Synthesegas beispielsweise Kohlenmonoxid und/oder Wasserstoff benötigen, nach wie vor überwiegend aus fossilen Quellen gespeist werden können. Zusätzlich dazu kann durch das Verfahren insbesondere durch die durch die zweite Empfangsstelle bereitgestellte dritte Menge an Reaktant, insbesondere während einer gesamten Betriebsdauer bzw. einer Betriebsdauer in einem hochgefahrenen Zustand der Anlage, eine gute Möglichkeit zur schrittweisen und ökonomisch tragfähigen Einführung grüner Technologien in die Synthese des Produkts erreicht werden.

In vorteilhafter Ausgestaltung der Erfindung wird die zweite Menge, welche aus der ersten Empfangsstelle geleitet wird und/oder die dritte Menge, welche aus der zweiten Empfangsstelle geleitet wird, jeweils derart begrenzt, sodass die in den Reaktionsbereich geleitete Summenmenge kleiner oder gleich der ersten Menge ist. Die erste Menge entspricht einer Ausbeute von 100 %, welche durch die Anlage in einem besonders vorteilhaften Betriebszustand erreicht werden kann. Somit wird durch die Reduzierung bzw. Begrenzung, insbesondere der zweiten Menge aber auch der dritten Menge gewährleistet, dass die Anlage nicht über ihrer bauartbedingten Ausbildung mit Reaktanten versorgt wird. Somit ergibt sich der Vorteil, dass die Anlage durch das Verfahren besonders effizient und gleichzeitig besonders im Hinblick auf durchzuführende Wartungsarbeiten wartungsarm betrieben werden kann.

In vorteilhafter Ausgestaltung der Erfindung wird die von der zweiten Empfangsstelle einleitbare dritte Menge nur begrenzt, wenn eine weitere Begrenzung der zweiten Menge, welche durch die erste Empfangsstelle empfangbar ist, diese derart verkleinert, dass die durch die Begrenzung begrenzte zweite Menge kleiner als eine Abnahmemenge wird, welche durch die erste Empfangsstelle zu leiten ist. Mit anderen Worten kann der Fall eintreten, bei welchem die Bereitstellungsmenge der zweiten Empfangsstelle so groß wird, dass die Summenmenge aus zweiter und dritter Menge größer ist als die erste Menge. Um wie bereits erwähnt die Anlage innerhalb eines besonders vorteilhaften Auslastungsbereichs und somit mit einem besonders großen Durchsatz bei gleichzeitig besonders geringer Ausfallwahrscheinlichkeit der Anlage betreiben zu können, sollte die Summenmenge reduziert werden. Dies kann beispielsweise für einen Zeitpunkt der Fall sein, bei welchem aufgrund besonders hoher Sonneneinstrahlung oder starken Windes eine besonders große Menge Reaktant als Bereitstellungsmenge an der zweiten Empfangsstelle verfügbar ist. Gleichzeitig kann es möglich sein, dass die Abnahmemenge des Reaktanten an der ersten Empfangsstelle technisch bedingt nicht weiter verringert werden kann, da sonst beispielsweise ein nicht erwünschtes Austreten des Reaktanten an der ersten Empfangsstelle zu erwarten ist, wird erst für diesen Fall die dritte Menge reduziert, sodass durch das Verfahren die erneuerbaren Energiequellen besonders vorteilhaft ausgenutzt werden können.

Entspricht beispielsweise die erste Menge 100 % Auslastung und es wird die zweite Menge in dem Maße in den Reaktionsbereich eingeleitet, dass die zweite Menge 80 % besteht, ist jedoch eine Bereitstellungsmenge welche eine Auslastung der Anlage von 30 % entspricht an der zweiten Empfangsstelle empfangbar und würde diese in Kombination mit der zweiten Menge als Summenmenge in den Reaktionsbereich geleitet werden würde die Summenmenge einer Auslastung der Anlage von 110 % entsprechen, was nicht gewünscht ist. So muss entweder die zweite Menge oder die dritte Menge reduziert werden, damit insgesamt eine Auslastung von 100 % entsprechende Menge an Reaktant in den Reaktionsbereich der Anlage geleitet wird. Vorteilhafterweise wird soweit möglich die zweite Menge, des durch fossile Energieträger hergestellten bzw. herzustellenden Reaktanten reduziert, sodass die dritte Menge, des durch erneuerbare Energiequellen hergestellten bzw. zur Verfügung gestellten Reaktanten besonders effizient in der Anlage verwendet werden kann.

Beispielsweise entspricht die Abnahmemenge 50 % der Auslastung der Anlage, welche aufgrund beispielsweise technischer Ausgestaltung der ersten Empfangsstelle nicht unterschritten werden kann. Die Abnahmemenge ist somit beispielsweise eine Mindestmenge fossil erzeugten Synthesegases, und bestimmt somit die geringste empfangbare zweite Menge. Wird beispielsweise von der ersten Empfangsstelle eine zweite Menge des Reaktanten, welche einer Auslastung von 80 % entspricht, eingeleitet. Steht nun an der zweiten Empfangsstelle zusätzlich eine dritte Menge, welche 60 % der Auslastung entspricht, bereit, könte die zweite Menge nur entsprechend um 30 % reduziert werden, um die Abnahmemenge, welche technisch bedingt an der ersten Empfangsstelle empfangen werden sollte, nicht zu unterschreiten. Daraufhin kann nun die dritte Menge um 10 % reduziert werden, damit insgesamt die erste Menge an Reaktant, welcher einer Auslastung von 100 % entspricht und dem Reaktionsbereich bereitgestellt bzw. in diesen geleitet oder gefördert wird, nicht überschritten wird.

In vorteilhafter Ausgestaltung der Erfindung ist die erste Empfangsstelle mit einer ersten Erzeugeranlage derart verbunden, dass die erste Empfangsstelle den Reaktant von der ersten Erzeugeranlage, welche zur Herstellung bzw. Erzeugung des Reaktanten oder dessen Bereitstellung ausgebildet ist, empfängt.

In weiterer vorteilhafter Ausgestaltung der Erfindung ist die zweite Empfangsstelle mit einer zweiten Erzeugeranlage derart verbunden, dass die zweite Empfangsstelle den Reaktant von der zweiten Erzeugeranlage, welche ebenfalls analog zu der ersten Erzeugeranlage dazu ausgebildet ist, den Reaktanten zu erzeugen bzw. bereitzustellen, empfängt. Durch das jeweilige Verbundensein der ersten Erzeugeranlage bzw. zweiten Erzeugeranlage mit der ersten Empfangsstelle bzw. zweiten Empfangsstelle kann der Reaktant bzw. die Reaktanten und somit der wenigstens eine Reaktant jeweils besonders vorteilhaft an der jeweiligen Empfangsstelle empfangbar sein, wodurch die Anlage besonders vorteilhaft betrieben werden kann.

In weiterer vorteilhafter Ausgestaltung der Erfindung wird in der ersten Erzeugeranlage der Reaktant aus einem ersten Ausgangsstoffgemisch erzeugt und/oder der Reaktant in der zweiten Erzeugeranlage aus dem ersten Ausgangsstoffgemisch und/oder aus einem weiteren Ausgangsstoffgemisch erzeugt. Das heißt in der jeweiligen Erzeugeranlage wird aus einem jeweiligen Stoffgemisch insbesondere durch das Zuführen von Energie, welche aus einer regenerativen und/oder einer fossilen Energiequelle bereitgestellt werden kann, der wenigstens eine Reaktant erzeugt. Dadurch ergibt sich der Vorteil, dass, insbesondere aufgrund der Wahl des Ausgangsstoffgemischs der Reaktant besonders vorteilhaft erzeugt werden kann.

In weiterer vorteilhafter Ausgestaltung der Erfindung wird die erste Erzeugeranlage und/oder die zweite Erzeugeranlage mit Energie aus einer regenerativen Energiequelle, wie beispielsweise Strom aus Sonnenenergie mittels beispielsweise einer Photovoltaikanlage, und/oder aus einer fossilen Energiequelle, wie beispielsweise Erdgas versorgt, um den Reaktanten zu erzeugen. Durch die Verwendung beispielsweise der regenerativen Energiequelle bzw. der erneuerbaren Energiequelle beispielsweise für die erste Erzeugeranlage und/oder der Verwendung fossiler Energiequellen für die zweite Erzeugeranlage kann auf besonders vorteilhafte Weise ein Beitrag für die Energiewende geleistet werden und darüber hinaus die Anlage besonders effizient betrieben werden.

In vorteilhafter Weise erfolgt die Erzeugung des wenigstens einen Reaktanten in der ersten Erzeugeranlage und/oder die Erzeugung des wenigstens einen Reaktanten in der zweiten Erzeugeranlage durch ein Elektrolyseverfahren und/oder durch eine Dampfreformierung und/oder durch eine, insbesondere umgekehrte, Wasser-Gas-Shift-Reaktion. Durch das Elektrolyseverfahren und/oder durch die Dampfreformierung und/oder durch die umgekehrte Wasser-Gas-Shift-Reaktion (RWGS-Reaktion), kann der Reaktant jeweils besonders vorteilhaft erzeugt werden, sodass die Anlage besonders effizient betrieben werden kann. Dabei können die Elektrolyse bzw. das Elektrolyseverfahren beispielsweise eine Wasserelektrolyse und/oder eine Kohlendioxidelektrolyse sein. So wird bei der Wasserstoffelektrolyse beispielsweise aus $2H_2O -> 2H_2 + O_2$ erzeugt, bei der Kohlendioxidelektrolyse kann Kohlendioxid in Verbindung mit Wasserdampf beispielsweise zu Kohlenmonoxid und Wasserstoff verwandelt werden, wobei es sich dabei um ein Gemisch handelt, welches insbesondere universell und insbesondere als Synthesegas für die Anlage eingesetzt werden kann. Das Synthesegas als Zwischenprodukt umfasst beispielsweise bereits die Elemente Kohlenstoff, Sauerstoff und Wasserstoff, welche beispielsweise bei dem Erzeugen des Produkts in Form von Methanol erforderlich sind.

In vorteilhafter Ausgestaltung umfasst ein Synthesegas den wenigstens einen Reaktant, welcher insbesondere Wasserstoff und/oder Kohlenmonoxid, aufweist, welches von der jeweiligen Empfangsstelle in den Reaktionsbereich geleitet wird. Dabei kann das Synthesegas der Reaktant sein bzw. das Synthesegas den Reaktanten umfassen oder mehrere Reaktanten das Synthesegas bilden. Durch die Verwendung von Synthesegas kann insbesondere aufgrund des gasförmigen Zustands des Synthesegases beispielsweise das Leiten des Reaktanten von der jeweiligen Empfangsstelle zu dem Reaktionsbereich beispielsweise besonders vorteilhaft erfolgen. Darüber hinaus kann die Reaktion bzw. die Synthese in dem Reaktionsbereich beispielsweise in der gasförmigen Phase des Synthesegases besonders vorteilhaft durchgeführt werden, sodass beispielsweise eine besonders geringe Menge an Katalysatormaterial, welches in den Reaktionsbereich eingebracht worden ist, verwendet werden kann.

In vorteilhafter Ausgestaltung des erfindungsgemäßen Verfahrens wird als das Produkt wenigstens ein Kohlenwasserstoff, insbesondere ein Kraftstoff wie beispielsweise Methanol erzeugt. Kohlenwasserstoffe sind Verbindungen, welche vielseitig verwendet werden können und insbesondere in Form von Kraftstoff als Energieträger dienen können, so kann durch das erfindungsgemäße Verfahren ein vielfältig einsetzbares Produkt gewonnen werden, sodass die Anlage besonders vorteilhaft eingesetzt werden kann. So könnte das erfindungsgemäße Verfahren auch für eine Anlage zur Ammoniaksynthese verwendet werden, wo kein Kohlenstoff sondern nur Wasserstoff und Stickstoff verwendet werden.

Weitere Vorteile, Merkmale und Einzelheiten der Erfindung ergeben sich aus der nachfolgenden Beschreibung eines bevorzugten Ausführungsbeispiels sowie anhand der Zeichnung. Bei dem im Folgenden erläuterten Ausführungsbeispiel handelt es sich um eine bevorzugte Ausführungsform der Erfindung. Bei dem Ausführungsbeispiel stellen die beschriebenen Komponenten der Ausführungsform jeweils einzelne, unabhängig voneinander zu betrachtende Merkmale der Erfindung dar, welche die Erfindung jeweils auch unabhängig voneinander weiterbilden und damit auch einzeln oder in einer anderen als der gezeigten Kombination als Bestandteil der Erfindung anzusehen sind. Des Weiteren ist die beschriebene Ausführungsform auch durch weitere der bereits beschriebenen Merkmale der Erfindung ergänzbar.

Dabei zeigt:

FIG 1     ein schematisches Ablaufdiagramm zum Betreiben einer Anlage, insbesondere einer Syntheseanlage, zur Synthese eines chemischen Produkts; und

FIG 2     einen schematischen Aufbau einer beispielhaften Anlage.

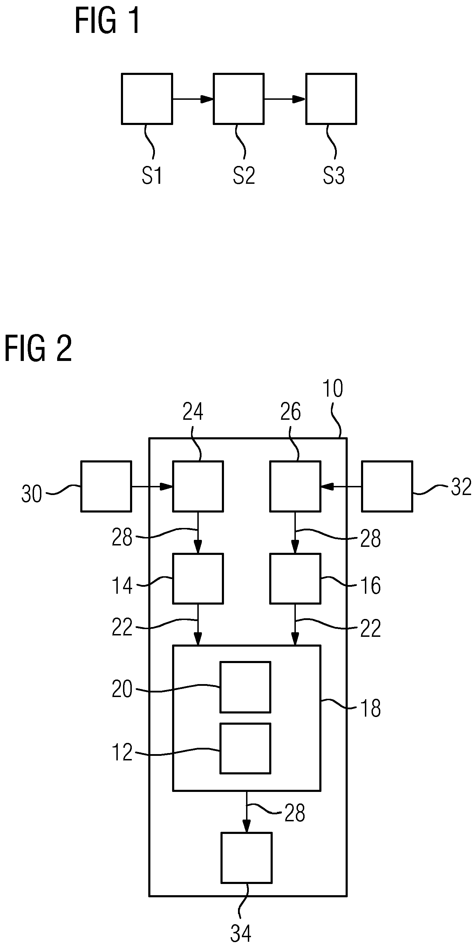

Die FIG 1 zeigt schematische ein Ablaufdiagramm für ein Verfahren zum Betreiben einer Anlage 10, welche beispielsweise insbesondere als Syntheseanlage für eine Methanolsynthese ausgebildet ist, wobei das Verfahren bzw. der Anlagebetrieb zur Synthese eines chemischen Produkts 12, im Ausführungsbeispiel insbesondere Methanol, dient.

Die Anlage 10 umfasst wenigstens zwei Empfangsstellen 14, 16, an welchen jeweils wenigstens ein Reaktant empfangbar ist, und wenigstens einen Reaktionsbereich 18 innerhalb welchem das Produkt 12 durch wenigstens eine Reaktion, vorteilhafterweise mittels eines Katalysators 20, aus dem wenigstens einen Reaktanten erzeugt wird. Dabei ist die Anlage 10 für einen bestimmten Durchsatz ausgelegt, welcher einer Auslastung von 100 % entspricht, bei welchem pro Zeiteinheit eine erste Menge an Reaktant, welche ebenfalls einer Auslastung von 100 % entspricht, in dem Reaktionsbereich 18 reagieren kann.

Damit mittels des Verfahrens die Anlage 10 besonders vorteilhaft betrieben werden kann, beispielsweise indem der wenigstens eine Reaktant, welcher an einer ersten der Empfangsstellen 14 empfangen werden kann und der Reaktant, welcher an einer zweiten der Empfangsstellen 16 empfangen werden kann, besonders vorteilhaft durch die Anlage 10 verarbeitet werden können, weist das Verfahren mehrere Schritte auf:

In einem ersten Schritt S1 des Verfahrens erfolgt ein Leiten einer zweiten gegenüber der ersten Menge geringeren Menge an Reaktant von der ersten Empfangsstelle 14 in den Reaktionsbereich 18, wobei das Leiten insbesondere kontinuierlich erfolgt, sodass ein kontinuierlicher Strom an Reaktant und somit ein Eduktstrom in den Reaktionsbereich eingeleitet werden kann.

In einem zweiten Schritt S2 des Verfahrens erfolgt ein Bestimmen einer durch den die zweite Empfangsstelle 16 empfangbaren Bereitstellungsmenge an Reaktant. Dabei entspricht die Bereitstellungsmenge der Menge, welche im Augenblick der Bestimmung bzw. in einem bestimmbaren Zeitintervall an der zweiten Empfangsstelle 16 abgerufen werden kann, somit entspricht die Bereitstellungsmenge einer maximal abrufbaren Menge an Reaktant zu einem bestimmten Zeitpunkt. Das bestimmen kann dabei mittels einer Mengenbestimmungsvorrichtung erfolgen.

In einem dritten Schritt S3 des Verfahrens erfolgt ein Leiten einer dritten Menge an Reaktant in Abhängigkeit von der Bereitstellungsmenge des Reaktanten von der zweiten Empfangsstelle 16 in den Reaktionsbereich 18, sodass eine Summenmenge umfassend die zweite Menge Reaktant aus der ersten Empfangsstelle 14 und die dritte Menge Reaktant aus der zweiten Empfangsstelle 16 in den Reaktionsbereich 18 geleitet wird.

Dabei erweist es sich als vorteilhaft, wenn das Verfahren dermaßen durchgeführt wird, dass die zweite Menge, welche von der ersten Empfangsstelle 14 geleitet wird, und/oder die dritte Menge, welche aus der zweiten Empfangsstelle 16 geleitet wird, jeweils derart begrenzt wird, sodass die Summenmenge kleiner oder gleich der ersten Menge ist, sodass insgesamt die Summenmenge an Reaktant in den Reaktionsbereich 18 geleitet wird. Dabei erfolgt die Begrenzung derart, dass vorteilhafterweise nur die von der ersten Empfangsstelle 14 eingeleitete zweite Menge begrenzt wird und die von der zweiten Empfangsstelle 16 einleitbare Menge nur begrenzt wird, wenn eine weitere Begrenzung der zweiten Menge die zweite Menge derart verkleinern würde, dass die begrenzte zweite Menge kleiner ist als eine Abnahmemenge, welche mindestens an der ersten Empfangsstelle 14 abgenommen werden sollte, um beispielsweise bauartbedingte Limitierungen einzuhalten. Das Leiten bzw. Fördern der jeweiligen Mengen kann vorteilhafterweise über Leitungen 22 erfolgen.

Vorteilhafterweise ist die erste Empfangsstelle 14 mit einer ersten Erzeugeranlage 24, beispielsweise über wenigstens eine weitere Leitung 28 derart verbunden, sodass die erste Empfangsstelle 14 den Reaktant von der ersten Erzeugeranlage 24 empfangen kann.

Vorteilhafterweise ist die zweite Empfangsstelle 16 mit einer zweiten Erzeugeranlage 26 derart verbunden, dass die zweite Empfangsstelle 16 den Reaktant von der zweiten Erzeugeranlage empfängt. Dabei kann die jeweilige Verbindung der ersten bzw. zweiten Erzeugeranlage 24, 26 mit der ersten bzw. zweiten Empfangsstelle 14 bzw. 16 über die weiteren Leitungen 28 erfolgen.

Bei dem wenigstens einen Reaktanten handelt es sich vorteilhafterweise um ein Synthesegas bzw. enthält das Synthesegas den wenigstens einen Reaktant, wobei das Synthesegas somit als Reaktant vorteilhafterweise insbesondere Wasserstoff und/oder Kohlenmonoxid aufweist.

Dabei wird der Reaktant beispielsweise in der ersten Erzeugeranlage 24 aus einem ersten Ausgangsstoffgemisch erzeugt, wobei die erste Erzeugeranlage 24 beispielsweise durch eine fossile Energiequelle 30 mit fossiler Energie zum Erzeugen des Reaktanten versorgt wird. Vorteilhafterweise wird die zweite Erzeugeranlage 26 in welcher der Reaktant aus dem ersten Ausgangsstoffgemisch und/oder aus einem von dem ersten Ausgangsstoffgemisch unterschiedlichen zweiten Ausgangsstoffgemisch erzeugt wird mit einer regenerativen Energiequelle 32 wie beispielsweise Wind oder Solarenergie betrieben bzw. mit Energie versorgt. Als Produkt 12 wird vorteilhafterweise ein Kohlenwasserstoff, insbesondere ein Kraftstoff, wie das Methanol, erzeugt.

Dabei kann die erste Erzeugeranlage 24, welches mittels der fossilen Energiequelle 30 mit Energie zum Erzeugen des Reaktanten versorgt wird beispielsweise den Reaktanten aus Dampfreformierung gewinnen. Dabei wird beispielsweise als Ausgangsstoffgemisch Erdgas, Leichtbenzin, Biomasse oder dergleichen verwendet, woraus Kohlenmonoxid, insbesondere als Reaktant, und Wasser Kohlendioxid und Wasserstoff gewonnen werden kann.

Im Ausführungsbeispiel kann die zweite Erzeugeranlage 26, welche insbesondere mit der regenerativen Energiequelle 32, welche eine besonders große durch das Verfahren kompensierbar Volatilität aufweisen kann, mit Energie versorgt wird, beispielsweise mittels eines Elektrolyseverfahrens oder einer umgekehrten Wasser-Gas-Shift-Reaktion (RWGS-Reaction) aus dem weiteren Ausgangsstoffgemisch den wenigstens einen Reaktanten erzeugen. Dabei kann es sich bei dem Elektrolyseverfahren beispielsweise um eine Wasserstoffelektrolyse oder eine Kohlendioxidelektrolyse handeln, wobei jeweils als Reaktant ein Synthesegas gewonnen wird bzw. als der wenigstens eine Reaktant ein Synthesegas gewonnen wird welches für die Reaktion zum Produkt Methanol, welches die Summenform $CH4_{40}$ aufweist enthält. Dabei ist das Synthesegas im weitesten Sinne ein Gasgemisch, das für eine Synthese bzw. eine Reaktion insbesondere zum Produkt, dem Methanol, eingesetzt wird.

Für die Methanolherstellung wird das Synthesegas auch als Methanolsynthesegas bezeichnet, welches beispielsweise aus Wasser und/oder Kohlendioxid durch Elektrolyse oder dergleichen gewonnen werden kann und in erster Linie vorteilhafterweise Kohlenmonoxid als Reaktant und Wasserstoff als Reaktant enthalten kann. Ferner könnte das Synthesegas beispielsweise aus fossilen Energieträgern durch chemische Umwandlung, wie Reformieren und/oder partielle Oxidation oder ähnliches, gewonnen werden.

Das in dem Reaktionsbereich 18 gewonnene Produkt 12 kann beispielsweise in einen Tank 34 gefördert bzw. geleitet werden, insbesondere ebenfalls über eine weitere Leitung 28.

Durch das gezeigte Verfahren kann die Anlage 10 derart betrieben werden, dass eine Erhöhung der Auslastung der, insbesondere als große Methanolsyntheseanlage ausgebildeten, Anlage 10 derart durchgeführt wird, dass vorwiegend aus der fossilen Energiequelle 30 beispielsweise durch Dampfreformierung in der Erzeugeranlage 24 gewonnener Reaktant verwendet wird und gleichzeitig eine Erniedrigung der Kapazität der Erzeugeranlage zur Synthesegaserzeugung derart gewählt wird, dass ein Fenster geschaffen wird. Dieses Fenster kann durch die kurzzeitige Produktion von Synthesegas aus der erneuerbaren Energiequelle 32 in der zweiten Erzeugeranlage 26 für die Erzeugung des Produkts vorteilhaft genutzt werden. Sowohl die Auslastung der Methanolsyntheseanlage (Anlage 10) als auch die der Erzeugeranlage 24 welche der Anlage 10 angegliedert ist werden also getriggert durch das Aufkommen an Synthesegas und somit Reaktant der erneuerbaren Energiequelle bzw. der zweiten Erzeugeranlage 26, welche im Ausführungsbeispiels ebenfalls der Anlage 10 zugeordnet ist. Die erste Erzeugeranlage 24 läuft vorteilhafterweise bei voller Auslastung, diese kann aber zu einem gewissen Teil reduziert werden, was vorteilhafterweise aufgrund der bestimmten Bereitstellungsmenge geschehen kann.

Die Methanolsynthese ist sowohl für einen kombinierten Einsatz beispielsweise der Kohlendioxidelektrolyse, welche als Verfahren durch die Erzeugeranlage 26 durchgeführt werden kann, als auch durch die Wasserelektrolyse, welche ebenfalls durch die Erzeugeranlage 26 durchgeführt werden kann, als auch bei der Kombination von Wasserelektrolyse und RWGS-Reaktion besonders geeignet. Bei der Methanolsynthese durch die Anlage 10 wird für einen hohen Umsatz bzw. eine hohe Produktausbeute zu Methanol also dem Produkt 12 vorteilhafterweise eine geringe Konzentration an Kohlendioxid ($CO_2$) ohnehin vorteilhafterweise verwendet. So kann ohne $CO_2$ die Kinetik der Umsetzung zu Methanol deutlich verlangsamt werden bzw. nicht stattfinden. Bei zu geringem $CO_2$-Anteil (kleiner 1 % $CO_2$ zu CO) im Reaktantenstrom, welcher in den Reaktionsbereich eingeleitet wird, kann besonders wenig Methanol erzeugt werden. Oberhalb von einem Verhältnis von 3 % $CO_2$ zu CO geht der Umsatz von Methanol ebenso wieder langsam zurück.

Somit ergibt sich für den Betrieb der Anlage 10 ein besonders vorteilhaftes Verhältnis beispielsweise zwischen 1 % und 3 % $CO_2$ zu CO für eine besonders hohe Ausbeute an Produkt 12. Sowohl die $CO_2$ bzw. Kohlendioxidelektrolyse als auch die besonders kostengünstige Ausführung der RWGS-Reaktion, insbesondere bei niedrigen Temperaturen, weisen einen gewissen Anteil an $CO_2$ im Synthesegas auf, der bei alleiniger Verwendung durch von der erneuerbare Energiequelle 32 bereitgestellten Synthesegas jedoch so hoch wäre, dass die Methanolsynthese durch die Anlage 10 nicht besonders effizient erfolgen könnte.

Durch die Einspeisung dieses "grünen", also mittels regenerativer Energie erzeugten, Reaktanten aus der zweiten Erzeugeranlage 26 mittels der Empfangsstelle 16 kann somit durch das Verfahren der Nachteil einer ansonsten aufgrund beispielsweise der Lastrampen nicht vorteilhaft mit einem Energiemix betreibbaren Anlage 10 umgangen werden, da durch das Verfahren eine besonders hohe Umsetzung des Produkts 12 erreicht werden kann. Durch das Verfahren können auch Nachteile kompensiert werden, welche bei der ausschließlichen Verwendung von grünem Synthesegas auftreten können, beispielsweise hohe Lastrampen und ein hoher Kohlendioxidanteil.

Somit ist durch das Verfahren eine besonders vorteilhafte Kombination für eine Nutzung bzw. einen Betrieb einer Anlage 10 wie beispielsweise einer konventionellen, insbesondere besonders großen Methanolsyntheseanlage möglich, da die bestehende Anlage 10 besonders vorteilhaft für die Verwendung erneuerbarer Energien in Kombination mit fossilen Energien verwendet werden kann. Zusätzlich kann durch das gezeigte Verfahren eine besonders lange Lebensdauer der Anlage 10 auch während einem Wandel, welchen die Energiewende mit sich bringt, erreicht werden, da der bestehende Reaktant als Rohstoff bzw. Eduktstrom vorteilhaft mit dem Reaktant der erneuerbaren Energien verwendet werden kann.

Alternativ zu dem gezeigten Verfahren wäre die Lösung der Einbindung der Anlage 10 in die Energiewende derart zu realisieren, dass die Anlage beispielsweise um Stromspeicher oder Gasspeicher erweitert werden müsste. Darüber hinaus könnte eine Lösung für die Probleme der Methanolsynthese, welche die Energiewende mit sich bringt in der Verwendung bzw. Entwicklung von neuen Reaktorkonzepten bzw. flexibleren Syntheseanlagen stehen, die deutlich höhere Lastrampen als die gezeigte Anlage 10 fahren könnten, welche somit zur alleinigen Nutzung von volatilen Energiequellen ohne fossile Grundlast ausgelegt sind. Das heißt neuartige Anlagen könnten derart entwickelt werden, dass sie einzig durch die Erzeugeranlage 26 mit Reaktant versorgt werden können. Darüber hinaus könnte eine Grundlast an Energie bzw. Strom aus der erneuerbaren Energiequelle 32 bereitgestellt werden, indem Wasserkraft verwendet wird.

Bezugszeichenliste

| | |
|---|---|
| 10 | Anlage |
| 12 | Produkt |
| 14 | erste Empfangsstelle |
| 16 | zweite Empfangsstelle |
| 18 | Reaktionsbereich |
| 20 | Katalysator |
| 22 | Leitung |
| 24 | erste Erzeugeranlage |
| 26 | zweite Erzeugeranlage |
| 28 | weitere Leitung |
| 30 | fossile Energiequelle |
| 32 | regenerative Energiequelle |
| 34 | Tank |
| S1 | erster Schritt |
| S2 | zweiter Schritt |
| S3 | dritter Schritt |

**Patentansprüche**

1. Verfahren zum Betreiben einer Anlage (10) zur Synthese eines chemischen Produkts (12), mit wenigstens zwei Empfangsstellen (14, 16), an welchen jeweils wenigstens ein Reaktant empfangbar ist, und wenigstens einem Reaktionsbereich (18), innerhalb welchen das Produkt (12) durch wenigsten eine Reaktion aus dem wenigstens einen Reaktanten erzeugt wird, wobei die Anlage (10) ausgelegt ist für einen bestimmten Durchsatz, bei welchem pro Zeiteinheit eine erste Menge an Reaktant in dem Reaktionsbereich (18) reagieren kann, mit den Schritten:

   - Leiten einer zweiten gegenüber der ersten Menge geringeren Menge an Reaktant von einer ersten der Empfangsstellen (14) in den Reaktionsbereich (18)(S1);
   - Bestimmen einer durch eine zweite der Empfangsstellen (16) empfangbare Bereitstellungsmenge an Reaktant (S2); und
   - Leiten einer dritten Menge an Reaktant in Abhängigkeit von der Bereitstellungsmenge des Reaktanten von der zweiten Empfangsstelle (16) in den Reaktionsbereich (18), sodass eine Summenmenge umfassend die zweite Menge Reaktant aus der ersten Empfangsstelle (14) und die dritte Menge Reaktant aus der zweiten Empfangsstelle (16) in den Reaktionsbereich (18) geleitet wird (S3).

2. Verfahren nach Anspruch 1, wobei die zweite Menge, welche aus der ersten Empfangsstelle (14) geleitet wird, und/oder die dritte Menge, welche aus der zweiten Empfangsstelle (16) geleitet wird, jeweils derart begrenzt wird, sodass die in den Reaktionsbereich (18) geleitete Summenmenge kleiner oder gleich der ersten Menge ist.

3. Verfahren nach Anspruch 2, wobei die von der zweiten Empfangsstelle (16) einleitbare dritte Menge nur begrenzt wird, wenn eine weitere Begrenzung der zweiten Menge diese derart verkleinert, dass die begrenzte zweite Menge kleiner als eine Abnahmemenge wird, welche durch die erste Empfangsstelle (14) zu leiten ist.

4. Verfahren nach einem der vorhergehenden Ansprüche, wobei die erste Empfangsstelle (14) mit einer ersten Erzeugeranlage (24) derart verbunden ist, dass die erste Empfangsstelle (16) den Reaktant von der ersten Erzeugeranlage (24) empfängt.

5. Verfahren nach einem der vorhergehenden Ansprüche, wobei die zweite Empfangsstelle (16) mit einer zweiten Erzeugeranlage (26) derart verbunden ist, dass die zweite Empfangsstelle (16) den Reaktant von der zweiten Erzeugeranlage (26) empfängt.

6. Verfahren nach Anspruch 4 oder 5, wobei in der ersten Erzeugeranlage (24) der Reaktant aus einem ersten Ausgangsstoffgemisch erzeugt wird und/oder der Reaktant in der zweiten Erzeugeranlage (26) aus dem ersten Ausgangsstoffgemisch und/oder aus einem weiteren Ausgangsstoffgemische erzeugt wird.

7. Verfahren nach Anspruch 6, wobei in der ersten Erzeugeranlage (24) und/oder in der zweiten Erzeugeranlage (26) der Reaktant mittels regenerativer Energie und/oder fossiler Energie erzeugt wird.

8. Verfahren nach Anspruch 6 oder 7, wobei die Erzeugung des Reaktanten in der ersten Erzeugeranlage (24) und/oder in der zweiten Erzeugeranlage (26) durch ein Elektrolyseverfahren und/oder durch eine Dampfreformierung und/oder durch eine umgekehrte Wasser-Gas-Shift-Reaktion erfolgt.

9. Verfahren nach einem der vorhergehenden Ansprüche, wobei ein Synthesegas den wenigstens eine Reaktant, welcher insbesondere Wasserstoff und/oder Kohlenmonoxid umfasst, aufweist, welches von der jeweiligen Empfangsstelle (14, 16) in den Reaktionsbereich (18) geleitet wird.

10. Verfahren nach einem der vorhergehenden Ansprüche, wobei als das Produkt (12) wenigsten ein Kohlenwasserstoff, insbesondere ein Kraftsoff, erzeugt wird.

...

# FIG 1

# FIG 2

Europäisches
Patentamt

European
Patent Office

Office européen
des brevets

## EUROPÄISCHER RECHERCHENBERICHT

**Nummer der Anmeldung**

EP 19 15 3501

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 2 464 617 A1 (SILICON FIRE AG [CH]) 20. Juni 2012 (2012-06-20) * Absätze [0030], [0045] - [0048], [0061] - [0069], [0075], [0076] * * Ansprüche 1,2,9,11 * ----- | 1-10 | INV. C01B3/34 C07C29/00 C25B1/00 C07C31/04 |
| X | DE 10 2010 028181 A1 (SIEMENS AG [DE]) 27. Oktober 2011 (2011-10-27) * Zusammenfassung * * Absätze [0004], [0007], [0009], [0024], [0025], [0030], [0032], [0036] * * Abbildung 1 * ----- | 1-10 | |
| A | DE 10 2013 102969 A1 (SUNFIRE GMBH [DE]) 25. September 2014 (2014-09-25) * Zusammenfassung * * Ansprüche 1,8 * ----- | 1-10 | |
| A | EP 2 998 385 A1 (HYENTEC GMBH [DE]) 23. März 2016 (2016-03-23) * Zusammenfassung * * Absätze [0004] - [0006], [0010], [0011], [0015], [0023] * ----- | 1-10 | RECHERCHIERTE SACHGEBIETE (IPC) C01B C07C C25B |

Der vorliegende Recherchenbericht wurde für alle Patentansprüche erstellt

| Recherchenort | Abschlußdatum der Recherche | Prüfer |
|---|---|---|
| München | 17. Juli 2019 | Alvarez Rodriguez, C |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
...............................................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 19 15 3501

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-07-2019

| Im Recherchenbericht angeführtes Patentdokument | Datum der Veröffentlichung | Mitglied(er) der Patentfamilie | Datum der Veröffentlichung |
|---|---|---|---|
| EP 2464617 A1 | 20-06-2012 | CA 2769950 A1<br>EP 2464617 A1<br>US 2012226080 A1<br>WO 2011018124 A1 | 17-02-2011<br>20-06-2012<br>06-09-2012<br>17-02-2011 |
| DE 102010028181 A1 | 27-10-2011 | DE 102010028181 A1<br>WO 2011134705 A1 | 27-10-2011<br>03-11-2011 |
| DE 102013102969 A1 | 25-09-2014 | KEINE | |
| EP 2998385 A1 | 23-03-2016 | DE 102014113388 A1<br>EP 2998385 A1 | 17-03-2016<br>23-03-2016 |

Für nähere Einzelheiten zu diesem Anhang : siehe Amtsblatt des Europäischen Patentamts, Nr.12/82